# EUROPEAN PATENT SPECIFICATION

(11) **EP 4 417 163 B1**
(45) Date of publication and mention of the grant of the patent: **20.05.2026**
(21) Application number: 24186366.1
(22) Date of filing: 28.08.2018
(51) Int. Cl.: A61B 17/04, A61F 2/08

(54) **SYSTEM FOR PREPARING A SOFT TISSUE GRAFT**
SYSTEM ZUR HERSTELLUNG EINES WEICHGEWEBETRANSPLANTATS
SYSTÈME DE PRÉPARATION D'UN GREFFON DE TISSU MOU

(30) Priority: 28.08.2017 US 201762550840 P; 19.09.2017 US 201762560379 P; 14.08.2018 US 201862718715 P
(43) Date of publication of application: 21.08.2024
(62) Divisional of application: 18773690.5
(73) Proprietor: CONMED Corporation, Largo, FL 33773 (US)
(72) Inventor: KAM, Andrew, Odessa (US); LOMBARDO, Giuseppe, New Port Richey (US)
(74) Representative: Strehl & Partner mbB

(56) References cited:
- EP-A1- 2 663 242
- US-A1- 2015 351 739
- US-A1- 2016 008 123
- US-A1- 2017 128 215

## Description

### CROSS-REFERENCE TO RELATED APPLICATIONS

This application claims priority to U.S. Provisional Patent Application Serial No. 62/550840, filed on August 28, 2017 and entitled "System for Preparing a Soft Tissue Graft," U.S. Provisional Patent Application Serial No. 62/560379, filed on September 19, 2017 and entitled "Soft Tissue Graft Preparation Suture Construct," and U.S. Provisional Patent Application Serial No. 62/718715, filed on August 14, 2018 and entitled "Graft Preparation System."

### BACKGROUND OF THE INVENTION

### 1. Field of the Invention

The invention relates generally to graft preparation and more particularly, a system for loading a suture construct on a soft tissue graft.

### 2. Description of Related Art

Grafting is a surgical procedure of transferring tissue from one surgical site to another surgical site. Grafting may be used in procedures to repair injuries to the skin, in dental implants, and in ligament repair, such as knee ligament repair, for example. As such, a variety of tissues may be grafted, including, but not limited to skin, bone, and tendons. Further, grafts can be composed of synthetic material or harvested from an individual (either the patient or a donor). A graft assembly from which the pre-characterizing part of claim 1 starts out is disclosed in US 2015/0351739 A1. Another conventional graft assembly is described in US 2016/008123 A1.

The ability to prepare grafts quickly and securely provides benefits to the surgeon in reducing the overall duration of the surgical procedure. One traditional method for preparing a graft includes whip stitching. Whip stitching is commonly used method for preparing a graft in ligament repair, such as ACL surgery. Further, during the step of harvesting a soft tissue autograft, it is desirable to pass stiches through the end to maintain control and provide counter traction. In both whip stitching and passing stiches during harvesting, the task of stitching the graft is a long and labor intensive process that requires multiple piercings of the graft. Every piercing increases time required to prepare the graft and causes trauma to the graft.

Other methods for preparing the graft have been developed to reduce trauma to the graft. For example, suture constructs having a fixed, central main line ("spine") have been deployed around the graft. The spine was designed to prevent collapsing of the suture construct prior to deployment around the graft. However, the spine limits the radial compression on the graft and reduces the contact area between the graft and the bone tunnel.

Therefore, there is a need for a system and method for graft preparation that reduces the number of piercings of the graft, reduces the time required to prepare the surgical graft, and improves the graft to bone contact.

### SUMMARY OF THE INVENTION

The present invention is directed to a graft assembly as set forth in the appended claims. The invention is useful for graft preparation, inter alia, in a system and method for loading a suture construct on a soft tissue graft.

The present invention is useful in a method for preparing a graft. The method does not form part of the claimed invention and comprises the steps of: (i) providing a frame having a proximal end and a distal end, a lumen extending through the frame from the proximal end to the distal end, a plurality of channels extending along the frame, and a transverse hole extending through the proximal end of the frame; (ii) inserting a pin into the transverse hole; (iii) inserting a graft into the lumen of the frame; and (iv) weaving a length of suture through the channels over the graft.

According to another aspect, the step of (iv) weaving a length of suture through the channels over the graft comprises the step of (v) threading a first tail and a second tail of the length of suture through a proximal end of the graft.

According to another aspect, the method includes the step of: (vi) removing the pin from the transverse hole.

According to yet another aspect, the method includes the step of: (vii) removing the frame from the graft with the woven length of suture from the frame.

According to another aspect, the method includes the step of: (viii) tensioning the first and second tails of suture.

According to another aspect, the step of (viii) tensioning the first and second tails of suture radially compresses the length of suture around the graft.

### BRIEF DESCRIPTION OF THE DRAWINGS

The foregoing and other objects, features, and advantages of the invention are apparent from the following description taken in conjunction with the accompanying drawings. Figures 1 to 3 and 8 to 11 do not represent embodiments of the invention.
FIG. 1 is a top perspective view schematic representation of a graft assembly in the open, unloaded configuration;
FIG. 2 is a top perspective view schematic representation of a graft assembly in the closed, loaded configuration;
FIG. 3 is a perspective view schematic representation of a graft in the prepared configuration;
FIG. 4 is a side view schematic representation of a trap of the graft assembly, according to an embodiment;
FIG. 5 is a perspective view schematic representation of a graft assembly in the closed, unloaded configuration, according to an embodiment.
FIG. 6 is a perspective view schematic representation of a graft assembly in the closed, loaded configuration, according to an embodiment;
FIG. 7 is a perspective view schematic representation of a graft in the prepared configuration, according to an embodiment;
FIG. 8 is a top perspective view schematic representation of a length of suture;
FIG. 9 is a top perspective view schematic representation of a suture construct;
FIG. 10 is a top perspective view schematic representation of a graft assembly in the closed, loaded configuration; and
FIG. 11 is a bottom perspective view schematic representation of a graft assembly in the closed, loaded configuration.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the scope of the appended claims will be apparent to those skilled in the art from this disclosure.

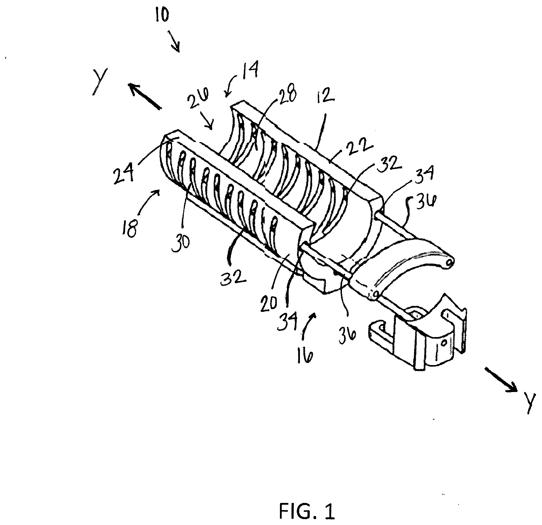

Referring now to FIG. 1, there is shown a top perspective view of a graft assembly 10 in the open, unloaded configuration. As shown, the graft assembly 10 comprises a frame 12 extending along a central longitudinal y - y axis. The frame 12 has an open side 14 extending along the central longitudinal y - y axis and extends between a proximal end 16 and a distal end 18. The frame 12 is semi-cylindrical such that the frame 12 comprises a wall 20 extending between a first edge 22 and second edge 24, defining a lumen 26 therebetween. The lumen 26 extends between the first and second edges 22, 24 through the frame 12.

As shown in FIG. 1, the wall 20 has a first side 28 extending to the first edge 22 on one side of the central longitudinal y - y axis and the wall 20 has a second side 30 extending to the second edge 24 on an opposing side of the central longitudinal y - y axis. First edge 22 and second edge 24 are parallel; however, other configurations of the frame 12 and the edges 22, 24 are contemplated. As also shown, the first and second edges 22, 24 are flat and extend in the same plane.

Still referring to FIG. 1, the wall 20 of the frame 12 comprises a plurality of channels 32 extending between the first edge 22 and the second edge 24. The channels 32 are paired such that two channels 32 converge near the central longitudinal y - y axis and diverge toward the first and second edges 22, 24. The frame 12 also comprises one or more transverse holes 34 on the proximal end 16 of the frame 12. The transverse holes 34 extend at least partially through the first and second sides 22, 24 of the frame 12. The transverse holes 34 extend through the first and second sides 22, 24 of the frame 12 to the distal end 18 and past all of the channels 32. The transverse holes 34 are sized and configured to receive pins 36. When the pins 36 are inserted into and extended through the transverse holes 34, the pins 36 retain the open lumen 26 and prevent inadvertent dislodging of the limbs of suture, as described in detail below. One or more pins 36 can be coupled in a variety of arrays and configurations to control the release of suture from the frame 12.

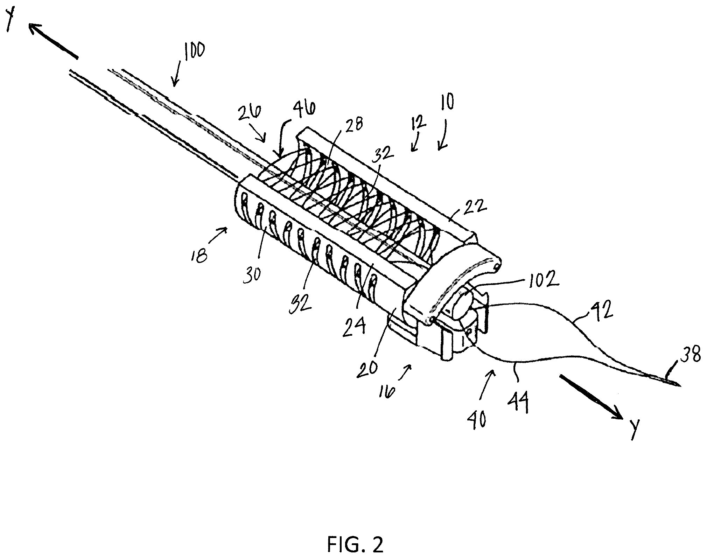

Turning to FIG. 2, there is shown a top perspective view schematic representation of a graft assembly 10 in the closed, loaded configuration. To achieve the closed, loaded configuration shown in FIG. 2, the pins 36 are first inserted into and extended through the transverse holes 34 on the proximal end 16 of the frame 12. Next, a graft 100 is loaded into the lumen 26 of the frame 12. As shown in FIG. 2, the graft 100 is loaded into the lumen 26 such that at least a proximal end 102 of the graft 100 extends beyond the wall 20 at the proximal end 16 of the frame 12.

Still referring to FIG. 2, after a graft 100 is inserted in to the lumen 26, a needle 38 is coupled to the proximal end 16 of the frame 12. The needle 38 is secured to the proximal end 16 of the frame 12 via suture 40. The needle 38 is then used to pierce through the graft 100, passing a first tail 42 and a second tail 44 of suture 40 (connected to the needle 38) therethrough. The suture 40 is passed through the graft 100 and through the channels 32. The arrangement or configuration of the channels 32 accepts the suture 40 into an opposing double helical pattern, forming a trap 46. When the trap 46 is formed, as shown in FIG. 2, the first and second tails 42, 44 of suture 40 are cut to remove the needle 38.

To deploy the trap 46 around the graft 100, all but one transverse pin 36 is removed from the frame 12. The trap 46 collapses on the graft 100 as tension is applied to the first and second tails 42, 44 of suture 40. By removing all but one transverse pin 36, the trap 46 collapses on the graft 100 but maintains the gapping or spacing (by the opposing double helical pattern). Once the trap 46 is deployed on the graft 100, the final transverse pin 36 is removed to free the graft 100, in the prepared configuration, from the frame 12.

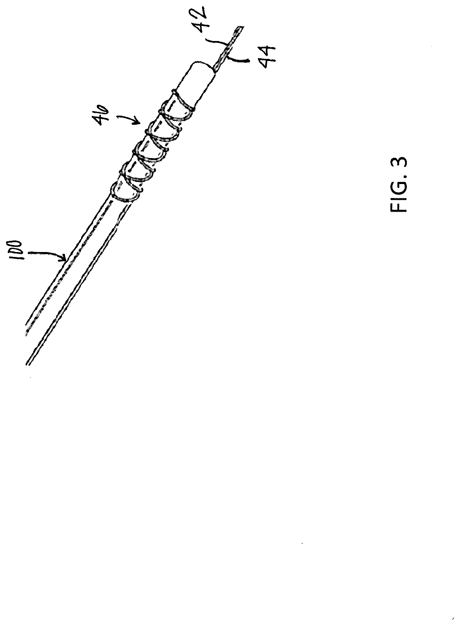

Referring now to FIG. 3, there is shown the graft 100 in the prepared configuration. As shown, the trap 46 is formed on the graft 100 in the opposing double helical pattern. Tension can be applied to the first and second tails 42, 44 of suture 40 to remove any remaining slack between the trap 46 and the graft 100. In the prepared configuration, any additional tension on the first and second tails 42, 44 of suture 40 causes radial compression (by the trap 46) on the graft 100.

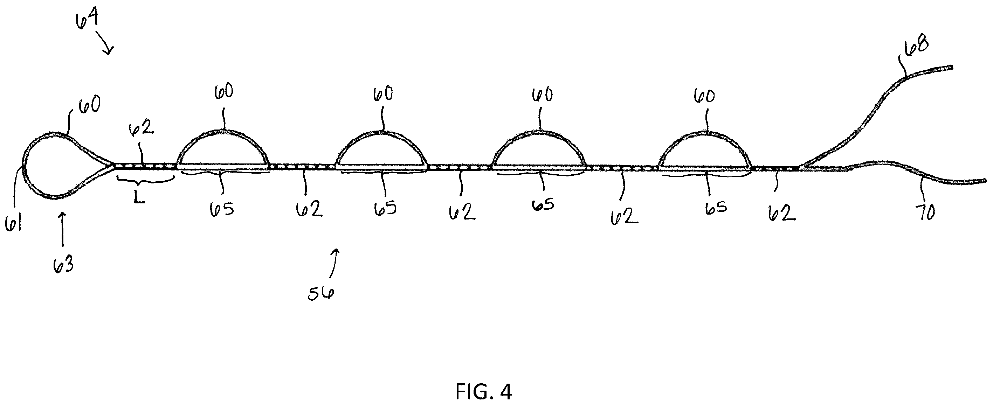

Turning now to FIGs. 4-7, there are shown perspective views schematic representations of a graft assembly 50, according to an embodiment. Referring now to FIG. 4, there is shown a perspective view schematic representation of a trap (e.g., suture construct) 64 of the graft assembly 50, according to an embodiment. The trap 64 shown in FIG. 4 is a suture construct composed of a single strand of suture 56. In the depicted embodiment, the suture 56 is folded at approximately the midpoint 61 of the suture 56 to form a closed end 63 extending to a first tail 68 and a second tail 70. The first tail 68 is spliced into the second tail 70, splice 62, over a first length L to create a closed loop 60 at the closed end 63.

Still referring to FIG. 4, additional splices 62 are formed by the first tail 68 into the second tail 70 in a series along the second tail 70, as shown. The number of splices 62 formed in the second tail 70 depends on a variety of factors, such as the size of the graft 100 and the surgical site for implantation of the graft 100, for example. In the depicted embodiment, there are gaps 65 between the splices 62, each of which forms a closed loop 60. Thus, there can be multiple closed loops 60 along the length of the graft 100.

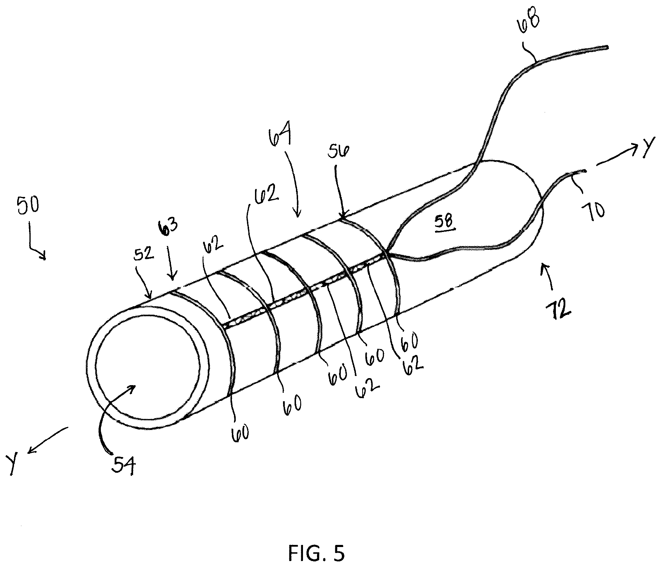

Turning now to FIG. 5, there is shown a perspective view of the graft assembly 50 in the closed, unloaded configuration, according to an alternative embodiment. The graft assembly 50 of FIG. 5 includes a hollow tube 52 extending along a central longitudinal y - y axis with an inner lumen 54. In the closed, unloaded configuration, the trap 64 (FIG. 4) is loaded onto the outer surface 58 of the hollow tube 52. In the depicted embodiment, the hollow tube 52 is inserted through each of the closed loops 60 with the splices 62 extending along the length of the hollow tube 52 of the trap 64. The formation of the closed loops 60 through multiple splices 62 functions to maintain the spacing of the closed loops 60. The splices 62 prevent collapsing of the trap 64 when tensioned around the graft 100, which can improve the contact between the graft 100 and a bone, as compared to other graft preparation methods. The splices 52 eliminate the need for a stationary or fixed spine extending along the length of conventional traps. In the embodiment depicted in FIGs. 5-7, when the trap 64 is loaded onto the hollow tube 52, the first and second tails 68, 70 of suture 56 extend from the splices 62 toward a proximal end 72 of the hollow tube 52.

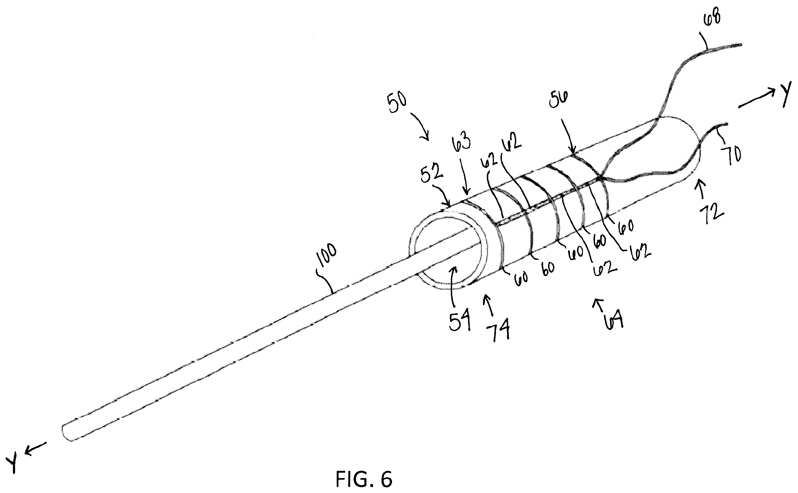

Referring now to FIG. 6, there is shown a perspective view schematic representation of the graft assembly 50 in the closed, loaded configuration, according to an embodiment. As shown in FIG. 6, a graft 100 is inserted into the inner lumen 54 of the hollow tube 52 through its distal end 74. The graft 100 can be any type of graft 100, including, but not limited to the soft tissue graft 100 shown in FIG. 3 and described above. To deploy the trap 64 around the graft 100, the hollow tube 52 is removed by pulling it back through the closed loops 60 of the trap 64.

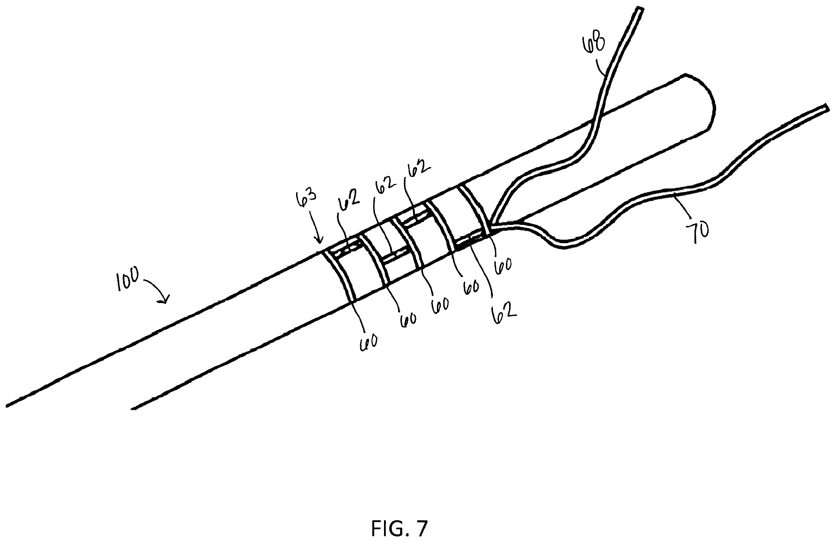

Turning now to FIG. 7, there is shown a perspective view schematic representation of the graft 100 in the prepared configuration, according to an embodiment. In the depicted embodiment, the hollow tube 52 has been pulled out from the closed loops 60 of the trap 64, leaving the trap 64 surrounding the graft. Once the trap 64 is around the graft 100, the first and second tails 68, 70 of suture 56 are pulled to tighten or otherwise compress the trap 64 around the graft 100, resulting in the prepared configuration shown in FIG. 7. As the first and second tails 68, 70 are tensioned, the splices 62 are tightened. As the slack in the splices 62 is reduced, the splices 62 may rotate (at least partially) around the graft 100, as shown in FIG. 7.

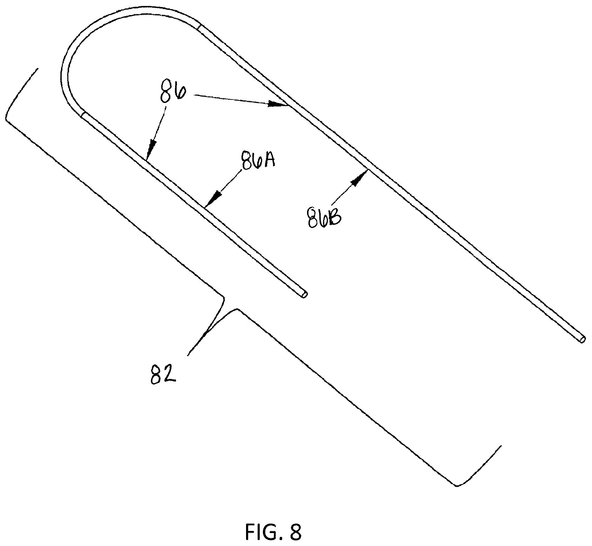

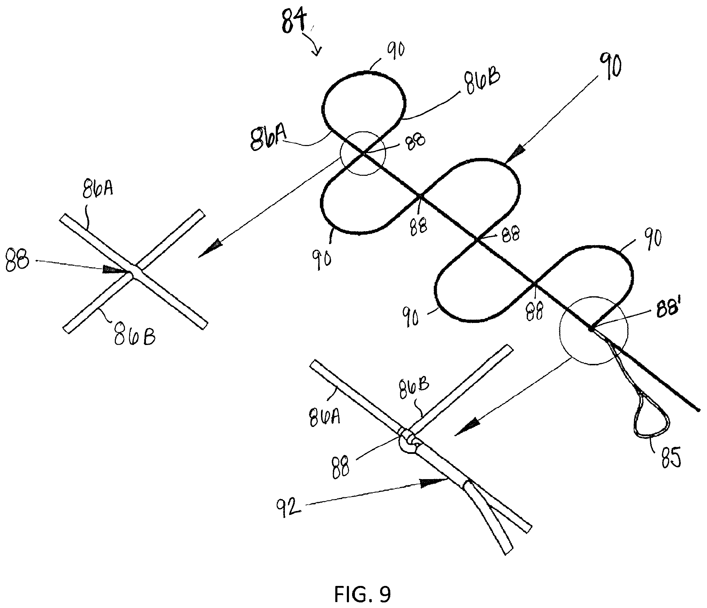

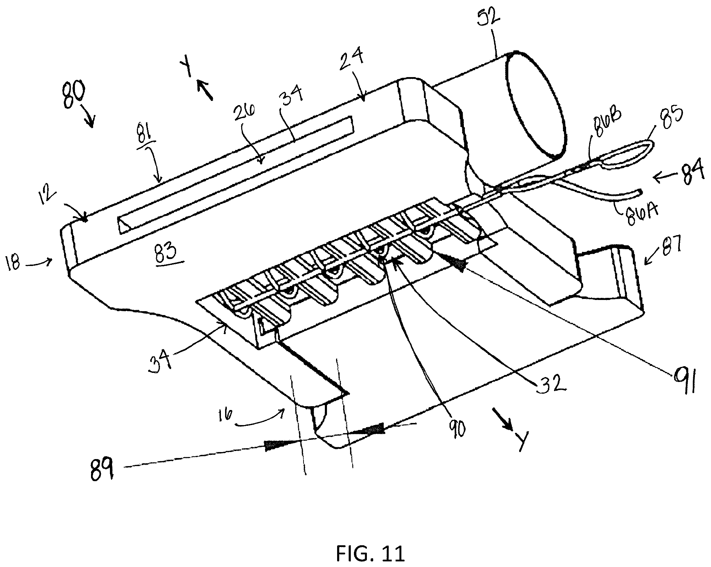

Turning now to FIGs. 8-11, there are shown perspective views schematic representations of a graft assembly 80. Referring now to FIG. 8, there is shown a top perspective view schematic representation of a continuous length of suture 82 used to create the trap (e.g., suture construct) 84 (shown in FIG. 9) of the graft assembly 80. The trap 84 (FIG. 9) is created by folding the length of suture 82 (i.e., into a "U" shape) forming two limbs 86, as shown in FIG. 8. Of the two limbs 86, there is a post, first limb 86A and a sliding, second limb 86B. The sliding, second limb 86B is pierced and passed through the post, first limb 86A at a series of passing locations 88, creating a series of adjustable loops 90 along the post, first limb 86A. Adjacent a last passing location 88', the sliding, second limb 86B is passed through a splice 92 in the post, first limb 86A, as shown in FIG. 9. The sliding, second limb 86B comprises a looped end 85, which extends from the splice 92.

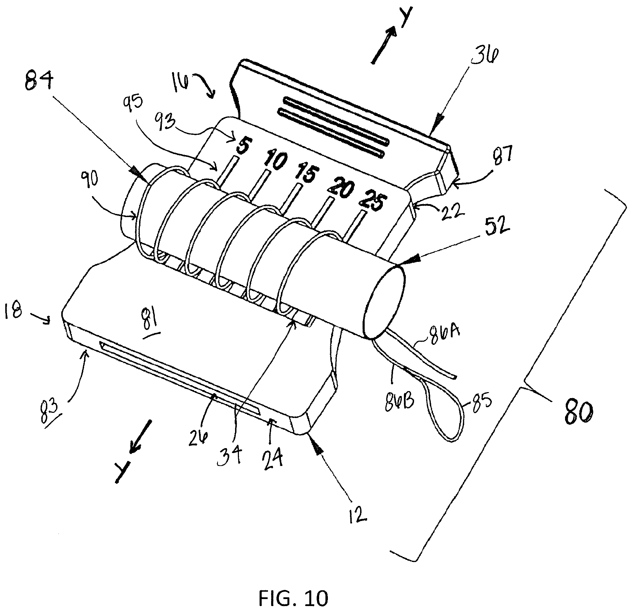

FIGs. 10-11 show top and bottom perspective views schematic representations of the trap 84 (in FIG. 9) loaded onto the graft assembly 80.

Similar to the graft assembly 10 in FIGs. 1-2, the graft assembly 80 in FIGs. 10-11 comprises a frame 12 extending along a central longitudinal y - y axis and between a proximal end 16 and a distal end 18. The frame 12 is substantially rectangular with a first side 22 at the proximal end 16 and a second side 24 at the distal end 18. A slot 26 extends from the second side 24 to a lumen 34 within the frame 12. The lumen 34 extends through a top side 81 and a bottom side 83 of the frame 12. The lumen 34 is sized or otherwise configured to receive a sliding spacer 36, which is comparable to the pins 36 of the graft assembly 100 shown in FIG. 1.

Still referring to FIGs. 10-11, the sliding spacer 36 is slidable within the lumen 34 of the frame 12. The sliding spacer 36 comprises a handle end 87, which is wider than the lumen 34 such that the handle end 87 facilitates proper placement of the sliding spacer 36 within the lumen 34. The sliding spacer 36 also comprises a pronged distal end with a plurality of fingers (e.g., rods or prongs) 91 connected thereto. When the graft assembly 80 is in the closed, loaded configuration, as shown in FIGs. 10-11, the sliding spacer 36 is inserted within the lumen 34 such that the fingers 91 extend across the lumen 34, creating a channel 32 between the fingers 91. The fingers 91 extend parallel to the central longitudinal y - y axis in the lumen 34.

To load the graft assembly 80, each loop 90 of the trap 84 is inserted through a channel 32 such that the post, first limb 86A extends on the bottom side 83 of the frame 12, maintaining the pitch 89 of the trap 84. As shown in FIGs. 10-11, the loops 90 extend up through the channels 32 and a hollow tube 52 is passed through the loops 90 on the top side 81 of the frame 12. When the trap 84 is loaded on the hollow tube 52, the trap 84 is in a collapsible helical shape. As shown, the hollow tube 52 extends transversely over the channels 32 (perpendicular to the central longitudinal y - y axis).

Similar to the embodiment shown in FIGs. 4-7, a graft (not shown) is inserted into the hollow tube 52 at a desired distance. The top side 81 of the graft assembly 80 has graduations 95 with indicators 93 denoting the distance the graft (not shown) has traveled within the hollow tube 52. Once the graft (not shown) is at a desirable position within the hollow tube 52, the hollow tube 52 is removed, leaving the graft (not shown) within the trap 84 on the top side 81 of the frame 12. Traction is then applied to the looped end 85 of the sliding, second limb 86B. The traction causes the helical shaped trap 84 to collapse radially around the graft (not shown). After the trap 84 is radially collapsed, the sliding spacer 36 is removed from the lumen 34 (and frame 12), releasing the trap 84. Additional tension can then be applied to completely collapse the trap 84 on the graft (not shown). The trap 84 shown in FIG. 9 reduced frictional resistance during the act of collapsing the trap 84 on the graft (not shown). The reduction in frictional resistance is due to the low sliding resistance of the sliding, second limb 86B passing through the post, first limb 86A at the passing locations 88. The terminal splice 92 resists loosening and maintains residual tension on the trap 84 after the tension on the sliding, second limb 86B is released.

## Claims

1. A graft assembly comprising a suture construct (64) having:
a closed end (63) and an open end;
a first tail (68) of suture and a second tail (70) of suture positioned adjacent to the open end;
a first closed loop (60) forming the closed end (63) that is formed from the first and second tails (68, 70) of suture;
a first splice (62) formed by the first tail (68) in the second tail (70) between the first closed loop (60) and the open end;
a second closed loop (60) formed from the first and second tails (68, 70) of suture between the first splice (62) and the open end; and
a second splice (62) formed by the first tail (68) in the second tail (70) between the second closed loop (60) and the open end,
**characterized in that** each splice (62) is formed by splicing the first tail (68) into the second tail (70) over a predetermined length (L) so that the splices (62) prevent collapsing of the suture construct (64) when tensioned around a graft (100).

2. The graft assembly of claim 1, further comprising a hollow tube (52) extending through the first closed loop (60) and the second closed loop (60).

3. The graft assembly of claim 2, wherein the first and second closed loops (60, 60) extend perpendicular to the central longitudinal axis extending through the hollow tube (52).

4. The graft assembly of claim 2 or 3, wherein the hollow tube (52) has an inner lumen (54) extending therethrough.

5. The graft assembly of claim 4, further comprising a graft (100) that extends at least partially through the inner lumen (54).

6. The graft assembly of any of claims 1 to 5, wherein the first and second tails (68, 70) of suture are of a single strand of suture (56).

## Patentansprüche

1. Transplantatanordnung mit einer Nahtkonstruktion (64), umfassend:
ein geschlossenes Ende (63) und ein offenes Ende;
ein erstes Ende (68) von Nahtmaterial und ein zweites Ende (70) von Nahtmaterial, die benachbart zu dem offenen Ende positioniert sind;
eine erste geschlossene Schlaufe (60), die das geschlossene Ende (63) bildet, das aus dem ersten und dem zweiten Ende (68, 70) von Nahtmaterial gebildet ist;
eine erste Spleißung (62), die durch das erste Ende (68) in dem zweiten Ende (70) zwischen der ersten geschlossenen Schlaufe (60) und dem offenen Ende gebildet ist;
eine zweite geschlossene Schlaufe (60), die aus dem ersten und dem zweiten Ende (68, 70) von Nahtmaterial zwischen der ersten Spleißung (62) und dem offenen Ende gebildet ist; und
eine zweite Spleißung (62), die durch das erste Ende (68) in dem zweiten Ende (70) zwischen der zweiten geschlossenen Schlaufe (60) und dem offenen Ende gebildet ist,
**dadurch gekennzeichnet, dass** jede Spleißung (62) durch Spleißen des ersten Endes (68) in das zweite Ende (70) über eine vorbestimmte Länge (L) gebildet ist, so dass die Spleißungen (62) ein Kollabieren der Nahtkonstruktion (64) verhindern, wenn sie um ein Transplantat (100) gespannt wird.

2. Transplantatanordnung nach Anspruch 1, ferner mit einem Hohlrohr (52), das sich durch die erste geschlossene Schlaufe (60) und die zweite geschlossene Schlaufe (60) erstreckt.

3. Transplantatanordnung nach Anspruch 2, wobei sich die erste und die zweite geschlossene Schlaufe (60, 60) senkrecht zu der zentralen Längsachse erstrecken, die sich durch das Hohlrohr (52) erstreckt.

4. Transplantatanordnung nach Anspruch 2 oder 3, wobei das Hohlrohr (52) ein inneres Lumen (54) aufweist, das sich dadurch erstreckt.

5. Transplantatanordnung nach Anspruch 4, ferner mit einem Transplantat (100), das sich zumindest teilweise durch das innere Lumen (54) erstreckt.

6. Transplantatanordnung nach einem der Ansprüche 1 bis 5, wobei das erste und das zweite Nahtmaterial-Ende (68, 70) von einem einzelnen Nahtmaterial-Strang (56) sind.

## Revendications

1. Ensemble de greffon comprenant une construction (64) de suture ayant :
une extrémité fermée (63) et une extrémité ouverte ;
une première queue (68) de suture et une deuxième queue (70) de suture positionnées adjacentes à l'extrémité ouverte ;
une première boucle fermée (60) formant l'extrémité fermée (63) qui est formée à partir des première et deuxième queues (68, 70) de suture ;
une première épissure (62) formée par la première queue (68) dans la deuxième queue (70) entre la première boucle fermée (60) et l'extrémité ouverte ;
une deuxième boucle fermée (60) formée à partir des première et deuxième queues (68, 70) de suture entre la première épissure (62) et l'extrémité ouverte ; et
une deuxième épissure (62) formée par la première queue (68) dans la deuxième queue (70) entre la deuxième boucle fermée (60) et l'extrémité ouverte,
**caractérisé en ce que** chaque épissure (62) est formée en épissant la première queue (68) dans la deuxième queue (70) sur une longueur (L) prédéterminée de telle sorte que les épissures (62) préviennent un effondrement de la construction (64) de suture lorsqu'elle est mise en tension autour d'un greffon (100).

2. Ensemble de greffon selon la revendication 1, comprenant en outre un tube creux (52) s'étendant à travers la première boucle fermée (60) et la deuxième boucle fermée (60).

3. Ensemble de greffon selon la revendication 2, dans lequel les première et deuxième boucles fermées (60, 60) s'étendent perpendiculaires à l'axe longitudinal central s'étendant à travers le tube creux (52).

4. Ensemble de greffon selon la revendication 2 ou 3, dans lequel le tube creux (52) a une lumière intérieure (54) s'étendant à travers celui-ci.

5. Ensemble de greffon selon la revendication 4, comprenant en outre un greffon (100) qui s'étend au moins partiellement à travers la lumière intérieure (54).

6. Ensemble de greffon selon l'une quelconque des revendications 1 à 5, dans lequel les première et deuxième queues (68, 70) de suture sont d'un unique brin (56) de suture.
